(19) Europäisches Patentamt
European Patent Office
Office européen des brevets

(11) **EP 3 532 840 B1**

(12) **EUROPÄISCHE PATENTSCHRIFT**

(45) Veröffentlichungstag und Bekanntmachung des Hinweises auf die Patenterteilung:
**11.09.2024   Patentblatt 2024/37**

(21) Anmeldenummer: **17786895.7**

(22) Anmeldetag: **17.10.2017**

(51) Internationale Patentklassifikation (IPC):
**G01N 33/18** (2006.01)    **G06N 3/08** (2023.01)
**G05B 13/00** (2006.01)    **G06F 17/18** (2006.01)

(52) Gemeinsame Patentklassifikation (CPC):
**G01N 33/18; G05B 13/027; G06N 3/088;**
G16H 20/10

(86) Internationale Anmeldenummer:
**PCT/EP2017/076461**

(87) Internationale Veröffentlichungsnummer:
**WO 2018/077666 (03.05.2018 Gazette 2018/18)**

(54) **VERFAHREN ZUR AUTOMATISIERTEN IN-LINE ERKENNUNG VON ABWEICHUNGEN EINES IST-ZUSTANDS EINES FLUIDES VON EINEM REFERENZZUSTAND DES FLUIDES AN HAND STATISTISCHER METHODEN, INSBESONDERE FÜR DIE ÜBERWACHUNG EINER TRINKWASSERVERSORGUNG**

METHOD FOR THE AUTOMATED IN-LINE DETECTION OF DEVIATIONS OF AN ACTUAL STATE OF A FLUID FROM A REFERENCE STATE OF THE FLUID ON THE BASIS OF STATISTICAL METHODS, IN PARTICULAR FOR MONITORING A DRINKING WATER SUPPLY

PROCÉDÉ DE DÉTECTION AUTOMATIQUE EN LIGNE D'ÉCARTS D'ÉTAT D'UN ÉTAT RÉEL D'UN FLUIDE PAR RAPPORT À UN ÉTAT DE RÉFÉRENCE DU FLUIDE À BASE DE MÉTHODES STATISTIQUE, EN PARTICULIER POUR LA SURVEILLANCE D'UNE ALIMENTATION EN EAU POTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.10.2016   DE 102016120663**

(43) Veröffentlichungstag der Anmeldung:
**04.09.2019   Patentblatt 2019/36**

(73) Patentinhaber:
• **Endress+Hauser Flowtec AG**
  **4153 Reinach (CH)**
• **Universität Basel**
  **4003 Basel (CH)**

(72) Erfinder:
• **PAGE, Rebecca**
  **4054 Basel (CH)**
• **HUGGENBERGER, Peter**
  **4143 Dornach (CH)**
• **WIESMEIER, Stefan**
  **82284 Grafrath (DE)**
• **WALDMANN, Daniel**
  **4147 Aesch (CH)**

(74) Vertreter: **Hahn, Christian et al**
**Endress+Hauser Group Services (Deutschland)AG +Co. KG**
**Colmarer Straße 6**
**79576 Weil am Rhein (DE)**

(56) Entgegenhaltungen:
WO-A1-2016/014610    WO-A1-2016/070195
US-A1- 2003 236 649    US-A1- 2009 292 195

• **PAGE REBECCA M ET AL: "Multivariate Analysis of Groundwater-Quality Time-Series Using Self-organizing Maps and Sammon's Map", WATER RESOURCES MANAGEMENT, SPRINGER NETHERLANDS, DORDRECHT, vol. 29, no. 11, 13 June 2015 (2015-06-13), pages 3957 - 3970, XP035520071, ISSN: 0920-4741, [retrieved on 20150613], DOI: 10.1007/S11269-015-1039-2**

• NIKOO MOHAMMAD REZA ET AL: "Water Quality Zoning Using Probabilistic Support Vector Machines and Self-Organizing Maps", WATER RESOURCES MANAGEMENT, SPRINGER NETHERLANDS, DORDRECHT, vol. 27, no. 7, 24 February 2013 (2013-02-24), pages 2577 - 2594, XP035375062, ISSN: 0920-4741, [retrieved on 20130224], DOI: 10.1007/S11269-013-0304-5

**Beschreibung**

Die Erfindung betrifft ein Verfahren zur automatisierten in-line Erkennung von Abweichungen eines Ist-Zustands eines Fluides von einem Referenzzustand des Fluides. Ferner betrifft die Erfindung eine zur Ausführung des Verfahrens ausgestaltete Vorrichtung.

In vielen Anwendungen der Prozess- und/oder Automatisierungstechnik wird anhand von Messgeräten, insbesondere anhand von in-line Messgeräten, der Zustand eines Fluides bestimmt und/oder überwacht, insbesondere hinsichtlich der Qualität des Fluides. Ein als in-line Messgerät bezeichnetes Messgerät erfasst dabei eine Messgröße direkt und unmittelbar in dem zu überwachenden Fluid, ohne das beispielsweise eine Entnahme oder Vorbehandlung einer Probe des Fluides erforderlich wäre. Eine Vielzahl derartiger Messgeräte wird von der Endress+Hauser Gruppe hergestellt und vertrieben.

Bei nicht-inline fähigen Messgeräten ist dagegen oftmals eine Probennahme durch einen Anwendungstechniker nötig, gegebenenfalls gefolgt von einer Vorbehandlung der zu analysierenden Probe, beispielsweise durch Zugabe von Reagenzien, die zu einer mit optischen oder elektrochemischen Sensoren erfassbaren Veränderung der Probe führen. Hierbei können im Vergleich zu den oben genannten in-line-Messmethoden zwar deutlich niedrigere Nachweis-/Bestimmungsgrenzen mit hoher Genauigkeit sowie selektiv, also auch bei Vorhandensein von Störsubstanzen, erreicht werden. Nachteilig ist jedoch die durch die Probennahme und -vorbehandlung erforderliche lange Messdauer und damit die einhergehende geringe Messfrequenz.

Es zeigt sich die folgende Problematik: Um unmittelbar Veränderungen des Fluides, insbesondere im Qualitätszustands d.h. bezüglich der Qualität des Fluides, detektieren zu können, ist die Verwendung von in-line Messgeräten eine notwenige Voraussetzung. Andererseits ist eine Bewertung anhand der mit den in-line Messgeräten erfassten Messwerte, beispielsweise in Bezug auf eine erhöhte Gefährdung aufgrund einer Kontaminationsquelle, zum Beispiel einer Havarie, oftmals schwierig. Es ist insbesondere sehr anspruchsvoll, eine potentielle Gefährdung der Fluidqualität anhand eines einzelnen in-line Messwerts (wie beispielsweise elektrische Leitfähigkeit, Temperatur oder pH Wert) eindeutig zu bestimmen.

Um dem zu begegnen, werden oftmals mit mehreren Messgeräten mehrere in-line Messwerte bezüglich unterschiedlicher Messvariablen im Wesentlichen gleichzeitig erfasst. Bei den Messvariablen handelt es sich zum Beispiel um unterschiedliche Messgrößen des Fluides und/oder um eine jeweils an unterschiedlichen Messstellen gemessene Messgröße des Fluides. Das Ziel ist dabei, dass anhand der Gesamtheit der in-line erfassten Messwerte bezüglich unterschiedlicher Messvariablen eine Aussage über den Zustand des Fluides hinsichtlich der Qualität des Fluides, insbesondere auch hinsichtlich der Veränderung des Zustands des Fluides über die Zeit, getroffen wird. Vor allem in komplexen Systemen ist trotz einer hohen Menge an Information, wie beispielsweise die Messwerte bezüglich unterschiedlicher Messstellen und -größen, eine rasche Evaluation des Ist-Zustands erforderlich.

Hierbei stellt sich damit die Aufgabe der kombinierten Auswertung der Messwerte bezüglich der Messvariablen. Es ist beispielsweise eine multivariate Datenanalyse der in-line erfassten Messwerte so vorzunehmen, dass unmittelbar und sicher unerwünschte Qualitätszustände des Fluides sowie dessen Veränderung erkannt werden. Dies gilt insbesondere für Anwendungsbereiche in denen eine hohe Sicherheit erforderlich ist. Ein im Rahmen der Anmeldung relevantes Beispiel ist die Bewertung von Roh- und/oder Trinkwasser, da eine Verminderung der Trinkwasserqualität, beispielsweise durch eine Kontaminierung mit pathogenen Keimen, sowohl unmittelbar als auch sicher erkannt werden sollte, um jegliche Risiken für die Bevölkerung auszuschließen.

Zur multivariaten Datenanalyse von Messwerten, insbesondere auch wiederkehrend erfassten Messwerten (auch sogenannten Zeitreihen) sind im Stand der Technik unterschiedliche Methoden bekannt. Ein Beispiel stellen neuronale Netze dar. Auf neuronalen Netzen basierende Methoden der multivariaten Datenanalyse sind mehrheitlich selbst-lernend ausgestaltet.

Eine Übersicht über eine auf neuronalen Netzen basierende Methode, insbesondere in Bezug auf die Bewertung von Roh- und/oder Trinkwasser ist in der Inauguraldissertation von R. Page beschrieben, welche an der Universität Basel im Jahre 2011 unter dem Titel "Approaches to Hazard-oriented Groundwater Management Based on Multivariate Analysis of Groundwater Quality" vorgelegt wurde. In dieser Arbeit wurde eine Möglichkeit vorgestellt, eine multivariate Datenanalyse in Hinblick auf die Überwachung von Trink- und/oder Rohwasser mittels neuronaler Netze vorzunehmen. Das Ergebnis ist gleichermaßen in dem wissenschaftlichen Artikel von R. Page et. al "Multivariate Analysis of Groundwater-Quality Time-Series Using Self-organizing Maps and Sammon's Mapping" im Jahre 2015 in der Zeitschrift "Water Resources Management" Ausgabe 29, Seiten 3957ff. veröffentlicht.

Das darin beschriebene Verfahren kombiniert eine spezielle Ausgestaltung neuronaler Netze, sogenannte selbstorganisierende Karten (auch Kohonenkarten oder englisch: "Self Organizing Maps", kurz SOM genannt) mit einer Sammon'schen Fehlerfunktion. Dabei führt eine Dimensionsreduktion zu einer Reduktion der Komplexität der Aufgabe der Früherkennung einer Gefährdung basierend auf in-line Messwerten, wobei der notwendige Informationsgehalt der Messwerte beibehalten wird.

Die im Wesentlichen gleichzeitig wiederkehrend erfassten Messwerte zu jedem Zeitpunkt $t_i$ werden dabei auf einen Vektor $x_{t_i}$ abgebildet, wobei die Sam-

mon'sche Fehlerfunktion E minimiert wird:

$$E = \frac{1}{\sum_{i<k} d_{ik}^*} \sum_{i<k} \frac{[d_{ik}^* - d_{ik}]^2}{d_{ik}^*}$$

Hierbei bezeichnen i und k zwei beliebige Zeitpunkte, wobei i=1,...,n und k=1,...,n die Anzahl der Zeitpunkte ist. Bei $d_{ik}^*$ handelt es sich um den Abstand in dem ursprünglichen Vektorraum zwischen dem Vektor xti der zu dem Zeitpunkt ti erfassten Messwerte und dem Vektor xtk der zu dem Zeitpunkt tk erfassten Messwerte. Bei $d_{ik}$ handelt es sich um den Abstand zwischen den dazugehörigen Vektoren in dem dimensionsreduzierten Vektorraum, basierend auf den SOM Berechnungen. Mittels der minimierten Sammon'schen Fehlerfunktion wird dabei eine Menge an dimensionsreduzierten Vektoren erhalten, die das ursprüngliche Muster der Messwertverteilung möglichst gut wiedergeben.

Die Kombination von SOM und der Sammon'schen Fehlerfunktion wird als SOM-SM bezeichnet. Die SOM-SM Methode ermöglicht es, die multivariaten Zeitreihen unter Beibehaltung der relativen Verteilung in Form von Vektoren darzustellen, die in einem dimensionsreduzierten Vektorraum angeordnet sind und die das Verhältnis der Messwerte zu allen Messzeitpunkten zueinander als Muster wiedergeben. Ausgehend von dieser Darstellung stellt sich die Aufgabe, ein konkretes Kriterium zur Bewertung des Fluides zu erhalten.

Der Erfindung liegt daher die Aufgabe zugrunde, ein sicheres und automatisierbares Verfahren zur in-line Überwachung eines Fluides, insbesondere eines sich in einem Prozess befindlichen Fluides, mittels eines neuronalen Netzes vorzuschlagen, wobei das Verfahren insbesondere Veränderungen hinsichtlich der Qualität rasch erfassen und evaluieren kann. Des Weiteren liegt der Erfindung die Aufgabe zugrunde, eine zum Verfahren ausgestaltete Vorrichtung zu erhalten.

Diese Aufgabe wird erfindungsgemäß gelöst durch ein Verfahren nach Anspruch 1 sowie durch ein Warnsystem nach Anspruch 14.

In Verfahrensschritt a) der Erfindung wird also zunächst ein Referenzdatensatz mittels des Referenzzustands erstellt. Der Referenzzustand ist dabei als ein als normal angesehener Zustand definiert, bei dem das Fluid in einem akzeptablen Zustand bezüglich Qualität vorliegt, d.h. alle Anforderungen an die Qualität des Fluides erfüllt sind. Der akzeptable Zustand kann zum Beispiel anhand einer für die Sicherheit kritischen, nicht-in-line gemessenen Messgröße validiert werden. In dem Referenzzustand liegt zum Beispiel weder eine Verunreinigungen des Fluides, noch ein außergewöhnlicher Betriebszustand vor. Innerhalb einer Zeitspanne, in der sich das Fluid in dem Referenzzustand befindet, werden dann wiederkehrend (d.h. zu verschiedenen Zeitpunkten tj) Messwerte bezüglich der in-line Messvariablen erfasst. Dabei werden zu jedem Zeitpunkt tj zumindest drei verschiedene Messvariablen im Wesentlichen gleichzeitig erfasst. Mittels der im Referenzzustand erfassten Referenzmesswerte wird also genau der Rahmen der Schwankungen abgebildet, welchem die Messvariablen natürlicherweise unterliegen und die hinsichtlich der Qualität akzeptiert werden.

Hierbei werden vorzugsweise die Zeitspanne und die Messfrequenz für die wiederkehrende Messwertnahme so gewählt, dass genügend Referenzmesswerte für die Messvariablen vorliegen. Dies hängt in der Regel von der Dynamik des Systems bezüglich betrieblichen und operationellen Zuständen ab, welcher das Fluid in dem Referenzzustand unterliegt, zum Beispiel um eine Sollwertkurve einer Füllung eines Reservoirs. Handelt es sich zum Beispiel bei der Dynamik um eine im Wesentlichen periodische Dynamik, sollten zumindest Messwerte innerhalb der Zeitspanne von einer Periode genommen werden. Für den Fall von Trink- und/oder Rohwasser ist häufig eine typische Zeitskala von ca. eine Woche ausreichend. Mit einer Messwertfrequenz von ca. 6 Messwerten pro Stunde liegen somit ca. 1000 Messzeitpunkte tj für die Referenzmesswerte vor.

Die erfassten Referenzmesswerte zu den n Zeitpunkten werden mit einem neuronalen Netz und anschließender Projektion in einen reduzierten Vektorraum dargestellt. Dadurch werden die zu n verschiedenen Zeitpunkte tj, j=1,...,n erfassten Referenzmesswerte auf jeweils einen Referenzvektor rtj abgebildet.

Anschließend werden zu einem Zeitpunkt ti inline Messwerte genommen, welche dann, anschließend zu der Anordnung in dem neuronalen Netzes und der Projektion, in Bezug zu den Referenzmesswerten gesetzt werden. Hierfür wird die Anordnung in dem neuronalen Netz und die Projektion in den Vektorraum der reduzierten Dimension d verwendet, wobei zu jedem Zeitpunkt ti ein neuronales Netz mit den Messwerten zu dem Zeitpunkt ti und mit den Messwerten zu allen dem Zeitpunkt ti vorangehenden Zeitpunkten (t1, ...,ti-1) gebildet wird. Die zu jedem Zeitpunkt ti erfassten Messwerte werden dabei auf einen Messvektor xti abgebildet.

Die reduzierte Dimension d ist dabei kleiner als die Anzahl der Messvariablen, im Fall von drei Messvariablen also zum Beispiel maximal d=2. Da die Dimension d und die Wahl der Messvariablen für die Messwerte und die Referenzmesswerte identisch ist, werden die Messwerte und die Referenzmesswerte in denselben Vektorraum projiziert.

Erfindungsgemäß wird zur Bewertung des Messvektors xti in Bezug zu der Menge aller n Referenzvektoren rtj in dem Verfahrensschritt f) der Kerndichteschätzer herangezogen:

$$p_h(xti) = \frac{1}{nh^d} \sum_{j=1}^{n} f\left(\frac{|xti - rtj|}{h}\right)$$

Der Kerndichteschätzer wird auch als Parzen-Fenster-Schätzer (englisch: Parzen-Window Density Estimation) bezeichnet. Dabei ist h zunächst eine vorgebbare Fensterbreite, und $f(\frac{|xti-rtj|}{h})$ eine Wahrscheinlichkeitsdichtefunktion, wobei als Argument der Wahrscheinlichkeitsdichtefunktion der betragsmäßige Unterschied zwischen dem Messvektor und dem Referenzvektor gebildet wird, geteilt durch die vorgebbare Fensterbreite h. Dies wird für jeden der n Referenzvektoren vorgenommen. Anschließend wird der Mittelwert gebildet, unter Berücksichtung der vorgebbaren Fensterbreite und der reduzierten Dimension d.

Der Kerndichteschätzer ist ein Maß dafür, wie wahrscheinlich das Auftreten des Messvektors xti angesichts der Gesamtheit der Referenzvektoren rtj, j=1 ,...,n ist. Hierbei wird diejenige Wahrscheinlichkeitsdichtefunktion eingesetzt, welche die fachkundige Person als Verteilung für die Referenzmesswerte zugrunde legen würde; auch dies hängt von dem jeweiligen Prozess bzw. der Dynamik des Fluides ab.

Die Vorteile der Erfindung sind die folgenden:

- Es kann eine automatisierte und rasche d.h. im Wesentlichen unmittelbare Bewertung anhand von in-line erfassten Messwerten vorgenommen werden.
- Zur Beurteilung des Zustands des Fluides, insbesondere hinsichtlich Qualität, müssen keine konkreten Grenzwerte bezüglich der in-line Messvariablen gewählt werden. Mittels des Referenzdatensatzes ist die natürliche Heterogenität des Fluides in dem als normal angesehenen Referenzzustand erfasst. Die Verwendung eines Referenzzustandes bedeutet, dass zuvor ungesehene Zustände und Schwankungen in Bezug auf die Qualität ohne weitere Angaben erfasst und beurteilt werden können.
- Im Gegensatz zu anderen auf neuronalen Netzen basierenden Methoden wird erfindungsgemäss das neuronale Netz mit den Referenzmesswerten einmal gebildet. Mit den Messwerten wird jeweils ein neuronales Netzes zu jedem Zeitpunkt ti neu gebildet. Somit wird die zeitliche Veränderung des Zustands in Bezug auf die Qualität sichtbar und mit der Historie in Beziehung gesetzt.

Ein neuronales Netz wird dabei mit der Gesamtheit aller n Referenzmesswerte gebildet. Für jeden Zeitpunkt ti wird ein weiteres neuronales Netz mit den wiederkehrend erfassten Messwerten neu gebildet und ein Messvektor xti erstellt. Bei der Normierung des Datensatzes der Messwerte vor der SOM Berechnung wird beispielsweise der berechnete Mittelwert des Datensatzes der Messwerte und die Standardabweichung des Referenzdatensatzes verwendet, um unterschiedliche Ausgangslagen (z.B. Saisonalität) und systemeigene Schwankungen mit zu berücksichtigen. Bei der Evaluation des Zustandes zum Zeitpunkt ti bezüglich Qualität wird der Messvektor zu dem Zeitpunkt ti mit der Menge aller Referenzvektoren verglichen. Bei dem erfindungsgemäßen Verfahren wird der Informationsgehalt optimiert, da sich das zweite neuronale Netz dem Datensatz der Messwerte anpassen kann und somit möglichst viel von der ursprünglichen Dynamik des Systems in den reduzierten Vektorraum übertragen wird. Die Referenzvektoren und die Messvektoren werden dabei unmittelbar anschließend zur in-line Erfassung der Messvariablen zum Zeitpunkt ti erstellt. Durch das erfindungsgemäße Verfahren wird daher eine im Wesentlichen permanente und adaptive in-line Überwachung des Ist-Zustands des Fluides, und dadurch letztendlich auch eine Bewertung eines Prozesses hinsichtlich der Fluidqualität ermöglicht.

In einer vorteilhaften Ausgestaltung erfolgt die Bildung des neuronalen Netzes und die Projektion auf Basis einer selbstorganisierenden Karte bzw. Kohonenkarte (SOM), wobei bei der Projektion die Sammon'schen Fehlerfunktion (SM) verwendet wird. Bezüglich dieser Ausgestaltung sei wieder auf den eingangs genannten wissenschaftlichen Artikel "Multivariate Analysis of Groundwater-Quality Time-Series Using Self-organizing Maps and Sammon's Mapping" verwiesen, in welchem die Ausführung der SOM-SM Methode detailliert beschrieben ist.

In einer Ausgestaltung ist die reduzierte Dimension d größer als eins, vorzugsweise zwei.

In einer bevorzugten Ausgestaltung ist die Wahrscheinlichkeitsdichtefunktion die Wahrscheinlichkeitsdichtefunktion einer Normalverteilung.

$$f\left(\frac{|xti - rtj|}{h}\right) = 2\pi^{-d/2} \cdot exp^{\frac{-(xti-rtj)^2}{2h^2}}$$

Selbstverständlich sind je nach Fluid und/oder Dynamik des Fluides auch andere aus dem Stand der Technik bekannte Wahrscheinlichkeitsdichtefunktionen möglich, wie beispielsweise die der Gammaverteilung, die der Cauchyverteilung bzw. Lorentzverteilung oder die der Weibullverteilung.

In einer Ausgestaltung wird die vorgebbare Fensterbreite mittels der Standardabweichung der Verteilung der Referenzmesswerte und/oder einem Abstand von Quantilen der Verteilung der Referenzmesswerte abgeschätzt.

Als Quantile werden im Stand der Technik verschiedene Bereiche der Verteilungen bezeichnet, wobei in jedem Bereich gleich große Anteile (=Quantil) der Verteilung liegen. Ein bekanntes Beispiel ist der Median, durch den die Werte der Verteilung in zwei umfangsgleiche Bereiche geteilt werden. Häufig werden auch Quartile verwendet, welche eine Verteilung in vier gleich große Teile zerlegen. Der Interquartilabstand (englisch: "interquartile range") bezeichnet die Differenz zwischen dem dritten und dem ersten Quartil, also Q (0,75) -Q (0,25). Der Interquartilabstand umfasst die mittleren 50 % der Verteilung und wird im Stand der Technik als Streuungsmaß verwendet.

Ein Beispiel ist, die vorgebbare Fensterbreite h des Kerndichteschätzers anhand des Interquartilabstands IQ4R und der Standardabweichung $\sigma$ zu schätzen:

$$h = 1.06 \cdot in \quad , \frac{IQ4R}{1.34} \Big\} \cdot n^{\frac{-1}{5}}$$

Selbstverständlich kann die vorgebbare Fensterbreite h auch nur durch den Interquartilabstand IQ4R oder einen anderen Quantilabstand oder die Standardabweichung bestimmt sein.

In einer Ausgestaltung erfolgt die Bewertung des Fluides zu dem Zeitpunkt ti hinsichtlich der Abweichung des Ist-Zustands vom Referenzzustand mittels einer Einteilung in zumindest zwei verschiedene Kategorien erster Art (Ka1 ,Ka2, ...). Die Einteilung hängt davon ab, ob der Kerndichteschätzer $p_h(xti)$ einen ersten oberen und/oder unteren vorgebbaren Grenzwert über-/unterschreitet. Bevorzugt sind ein oberer und ein unterer Grenzwert vorhanden.

In einer bevorzugten Ausgestaltung der Erfindung wird bei der Bewertung des Ist-Zustands des Fluides für den Zeitpunkt ti der Unterschied zwischen dem Messvektor xti mit einem zweiten Messvektor xtk berücksichtigt. Der zweite Messvektor xtk gehört zu einem dem Zeitpunkt ti vorangehenden Zeitpunkt tk, d.h. k=1,...,i-1. Bei dem Zeitpunkt tk kann es sich also zum Beispiel um den dem Zeitpunkt ti unmittelbar vorangehenden Zeitpunkt ti-1 handeln.

In dieser Ausgestaltung wird also zusätzlich zu der Bewertung anhand des Kerndichteschätzers noch die Historie des Systems berücksichtigt. Die Bewertung des Fluides zu dem Zeitpunkt ti erfolgt also einerseits im Hinblick auf die Wahrscheinlichkeit des Auftretens der Messwerte zu dem Zeitpunkt ti bezüglich der Referenzmesswerte (Kerndichteschätzer-Kriterium), andererseits im Hinblick auf die Entwickelung bzw. Historie der Messwerte, so dass zum Beispiel sprunghafte Änderungen erkannt werden können. Zur Bewertung des Unterschieds wird zum Beispiel der Abstand d_ik der Messvektoren xti und xtk verwendet, d.h. d_ik=|xti-xtk|.

In einer Weiterbildung dieser Ausgestaltung erfolgt die Bewertung des Fluides zu dem Zeitpunkt ti hinsichtlich der Abweichung mittels einer Einteilung in zumindest zwei verschiedene Kategorien zweiter Art. Die Einteilung in die zumindest zwei Kategorien zweiter Art hängt davon ab, ob der Unterschied zwischen dem Messvektor xti und dem zweiten Messvektor xtk einen oberen und/oder unteren vorgebbaren Grenzwert über-/unterschreitet.

In einer besonders vorteilhaften Weiterbildung wird ein Warnsystem mit zumindest zwei Warnstufen zur Anzeige der Abweichung des Ist-Zustands vom Referenzzustand und zur Anzeige des Unterschiedes zwischen dem Messvektor xti und dem Messvektor xtk verwendet. Das Warnsystem basiert dabei auf einer Kombination der Kategorien erster und zweiter Art. Ein derartiges Warnsystem wurde auch in der Fachveröffentlichung von R. Page und P. Huggenberger in der Zeitschrift "Aqua & Gas" N°12, Seite 28ff im Jahre 2015 vorgestellt, ohne jedoch die erfindungsgemäßen konkreten Kriterien zur Berechnung der Bewertungskriterien anzugeben.

Im einfachsten Fall handelt es sich um eine Linearkombination mit gleich oder unterschiedlich gewichteten Linearkoeffizienten. Eine andere Möglichkeit sind aber auch nicht-lineare Kombinationen. Zum Beispiel kann man den Einfluss des Kerndichteschätzers (Kategorie erster Art) oder den des Unterschieds in Bezug auf die Historie (Kategorie zweiter Art) mit einer Exponentialfunktion in das Warnsystem einfließen lassen, je nachdem, ob das Warnsystem als empfindlich auf Abweichungen des Ist-Zustands vom Referenzzustand oder auf die Historie des Fluides ausgelegt werden soll.

In einer Weiterbildung zeigt das Warnsystem zum Zeitpunkt ti den Beitrag jeder Messvariable zu der Warnstufe an.

In einer weiteren Weiterbildung zeigt das Warnsystem zum Zeitpunkt ti den Beitrag der Kategorien erster und zweiter Art zu der Warnstufe an.

Gegebenenfalls werden in dieser Weiterbildung nur für den Fall, dass eine erhöhte Warnstufe vorliegt, die Beiträge der Messvariablen und/oder der Kategorien erster und zweiter Art angezeigt.

In einer Ausgestaltung des erfindungsgemäßen Verfahrens wird der Verfahrensschritt a) für zumindest zwei unterschiedliche

Referenzzustände des Fluides durchgeführt. Vor der Durchführung der Verfahrensschritte c)-f) wird der folgende Verfahrensschritt a2) durchgeführt:

a2) es wird einer der zumindest zwei unterschiedlichen Referenzzustände ausgewählt.

Die Verfahrensschritte c)-f) werden dann anschließend zum Verfahrensschritt a2) in Bezug zu dem jeweils ausgewählten Referenzzustand durchgeführt. Dies bedeutet, dass für die Bewertung mittels des Kerndichteschätzers in Schritt f) die Messwerte nur mit den Referenzmesswerten des jeweils ausgewählten Referenzzustands verglichen werden.

In einer Weiterbildung handelt es sich bei dem Fluid um Roh- und/oder Trinkwasser. Das Roh- und/oder Trinkwasser kann beispielsweise aus einem Trinkwasserzwischenspeicher, einer Wasserfassung, einem Grundwasserleiter oder einer Quelle stammen. Das Roh- und/oder Trinkwasser kann sich in einem Behälter wie etwa in einem Tank, Speicher einer Rohrleitung, einem Beobachtungsrohr und/oder einem Grundwasserbrunnen befinden. Die eingangs definierten Messstellen können aber auch in verschiedenen Brunnen oder Beobachtungsrohren von unterschiedlicher Tiefe in der Nähe voneinander sein, wobei die Messstellen (zum Beispiel durch Wasserläufe und/oder -leitungen) miteinander kommunizieren.

In einer Weiterbildung dieser Ausgestaltung sind die zwei unterschiedlichen Referenzzustände dadurch definiert, ob das Roh- und/oder Trinkwasser einem Pumpbetrieb unterliegt oder nicht. Der Pumpbetreib liegt z.B. im Falle der Grundwasserförderung vor.

In einer weiteren Weiterbildung sind die zumindest zwei unterschiedlichen Referenzzustände dadurch definiert, ob das Roh- und/oder Trinkwasser von unterschiedlichen Quellen, Seewasseranlagen und/oder Grundwasserleitern stammt.

In einer Ausgestaltung des Verfahrens sind die Messgrößen ausgewählt aus der Gruppe der folgender Messgrößen:

- Füllstand, Temperatur, Trübung, Sauerstoffgehalt, elektrische Leitfähigkeit, Druck, Redoxpotential, Durchfluss, pH-Wert, spektraler Absorptionskoeffizient.

Prinzipiell eignen sich selbstverständlich auch andere aus dem Stand der Technik bekannte Messgrößen, insbesondere Analysemessgrößen, welche in-line gemessen werden können.

Bezüglich der Vorrichtung wird die Aufgabe durch Anspruch 17 gelöst. Anspruch 17 umfasst ein Warnsystem zur automatisierten in-line Erkennung von Abweichungen eines Ist-Zustands eines Fluides von einem Referenzzustand des Fluides. Die Vorrichtung umfasst eine Messanordnung aufweisend zumindest drei in-line Messgeräte. Außerdem umfasst die Vorrichtung ein Computerprogrammprodukt. Die in-line Messgeräte und das Computerprogrammprodukt sind dazu ausgestaltet, das erfindungsgemäße Verfahren auszuführen.

In einer Weiterbildung ist das Computerprogrammprodukt dazu ausgestaltet, das Speichern und/oder die Verarbeitung der Referenzmesswerte und/oder der Messwerte zumindest teilweise auf zumindest einem Server und/oder in einer Cloud auszuführen.

Zusammenfassend können mittels der Erfindung Veränderungen in einem sich in einem Prozess befindlichen Fluides rasch und unmittelbar erkannt, bewertet und angezeigt werden. Die Veränderung bezieht sich in den Ausführungsbeispielen auf das Gefährdungspotential eines Zustands hinsichtlich der Qualität, indem Abweichungen eines Ist-Zustands eines Fluides von einem Referenzzustand des Fluides und Veränderungen des Zustands des Fluides hinsichtlich Qualität evaluiert werden. Abschließend sei angemerkt, dass die Erfindung nicht auf die systematische Überwachung der Qualität von Wasser beschränkt ist. Andere Anwendungsbeispiele umfassen beispielsweise die Beurteilung von Fluiden in Prozessen der chemischen und/oder pharmazeutischen, oder der Lebensmittel-verarbeitenden Industrie.

Die Erfindung wird anhand der nachfolgenden Figuren näher erläutert. Es zeigt:

Fig. 1: Eine Ausgestaltung der erfindungsgemäßen Vorrichtung.

Fig. 2: Eine Ausgestaltung des erfindungsgemäßen Verfahrens.

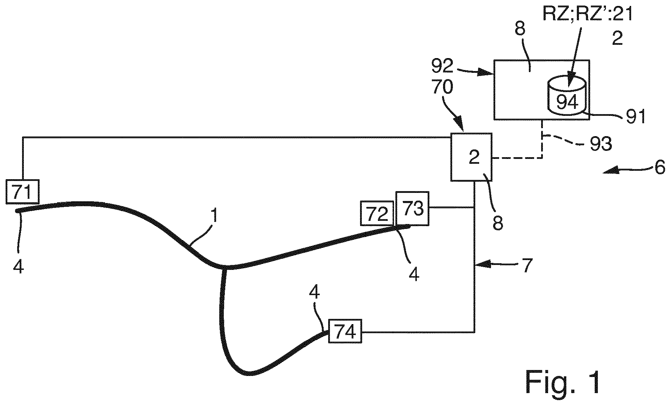

Fig. 1 zeigt eine schematische Darstellung einer Ausgestaltung einer erfindungsgemäßen Vorrichtung. Hierbei ist das Fluid 1 als ein Wasserlauf mit drei unterschiedlichen Messstellen 4 dargestellt. Die Messanordnung 7 umfasst dabei vier verschiedene in-line Messgeräte 71,72,73,74, welche die Messvariablen MV1,MV2,MV3,MV4 im Wesentlichen gleichzeitig erfassen. Die Messanordnung 7 umfasst in diesem Beispiel auch eine übergeordnete Einheit 70, an welche die Referenzmesswerte 21 und die Messwerte 2 übertragen werden. Das Fluid 1 befindet sich zunächst in einem Referenzzustand RZ, wobei die Referenzmesswerte 21 in einem Referenzzeitraum (zum Beispiel eine Woche) erfasst werden. Die Erfindung ist selbstverständlich keineswegs auf das hier gezeigte Ausführungsbeispiel beschränkt.

Anschließend werden die Referenzmesswerte 21 von einem Computerprogrammprodukt 8 über eine Datenverbindung 93 an eine Datenbank 94 übermittelt. Die Datenverbindung 93 kann zum Beispiel als eine Internetverbindung ausgestaltet sein, aber auch als jede andere aus dem Stand der Technik bekannte Datenverbindung. Die Datenbank 94 kann sich auf einem Server 91 befinden, wobei der Server wiederum zumindest teilweise Teil einer Cloud 92 sein kann. Auch die Messwerte 2 werden anhand der Datenverbindung 93 an die Datenbank 94 übermittelt. Das Computerprogrammprodukt 8 erstellt dabei die Bewertung anhand der Referenzmesswerte 21, der Messwerte zum Zeitpunkt ti sowie den Wert des Kerndichteschätzers. Dies wird für mehrere Zeitpunkte ti wiederkehrend regelmäßig durchgeführt.

Die Vorrichtung kann dabei Teil einer dezentralen Qualitätsüberwachung sein, zum Beispiel in einem sogenannten "Software as a Service" (kurz SaaS) Model, wobei nur die Warnstufen WO,W1,W2 übermittelt und angezeigt werden. Hierbei zeigt die Warnstufe WO zum Beispiel "keine Gefährdung" an, während die Warnstufe W1 "leicht erhöhte Gefährdung " und die Warnstufe W2 "hohe Gefährdung " anzeigt. Anhand der Warnstufen WO,W1,W2 werden somit Veränderungen im Wesentlichen unmittelbar d.h. on-line im Rahmen eines dezentralen 24h-Überwachungssystems übermittelt, wobei die Überwachung anhand von in-line Messgeräten 71,...,74 erfolgt. Dabei wird zum Beispiel nur für den Fall, dass die Warnstufen W1 oder W2 vorliegen, der jeweilige Beitrag der Messvariablen MV1,MV2,MV3 zu der Warnstufe W1 oder W2 angezeigt. Liegt zum Beispiel die Warnstufe W1 vor und der Beitrag von der Messvariable MV1 zu der Warnstufe W1 ist im Wesentlichen 100% oder knapp 100%, kann zum Beispiel zunächst das der Messvariable MV1 zugeordnete Messgerät 72 an der jeweiligen Messstelle 4 überprüft werden. Hiermit wird sowohl die eingangs erwähnte Sicherheit gegeben, aber auch das Auftreten von Fehlalarmen vermieden.

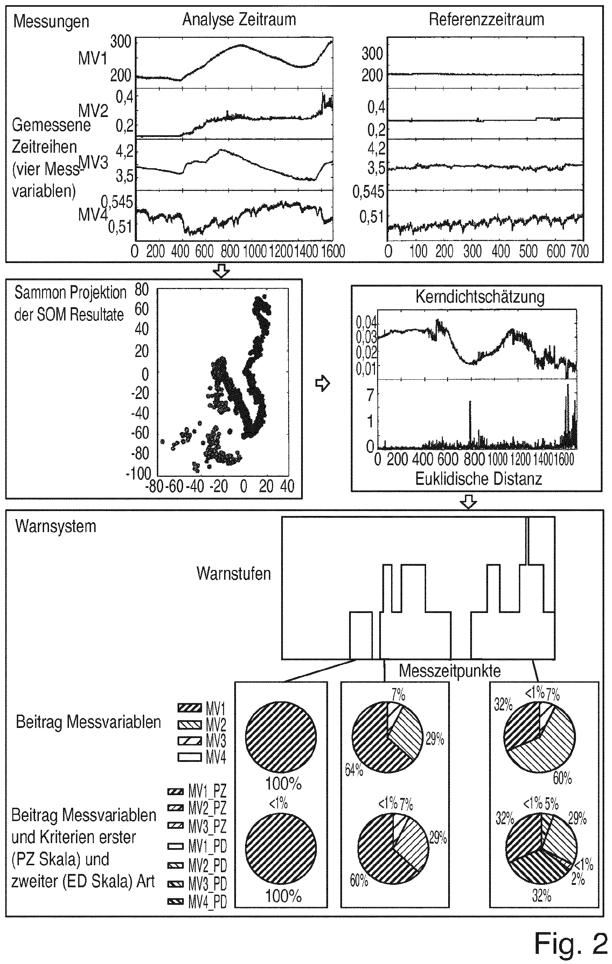

In Fig. 2 ist ein Schema einer Ausgestaltung des erfindungsgemäßen Verfahrens dargestellt. In dieser Ausgestaltung ist für den Fall von vier Messvariablen MV1,MV2,MV3,MV4 die wiederkehrende Erfassung der Messwerte 2 zu den Zeitpunkten ti in dem Analysezeitraum, sowie die wiederkehrende Erfassung der Referenzmesswerte 21 zu den Zeitpunkten tj in dem Referenzzeitraum dargestellt. Der Referenzzeitraum beträgt in dem Beispiel ca. eine Woche, in welcher das Fluid in einem Referenzzustand RZ vorliegt. In einer anderen Ausgestaltung können auch mehrere derartige Zeitreihen für die Messvariablen MV1,MV2,MV3,MV4 vorliegen, jeweils für einen ersten Referenzzustand RZ und einen weiteren Referenzzustand RZ'.

In einem automatisierten Verfahren wird zunächst das neuronale Netz mit den Referenzmesswerten 21 erstellt wobei es sich bei dem neuronalen Netz um eine SOM oder Kohonenkarte handelt. Anhand der Sammon'schen Projektion (SM) der SOM Resultate wird die SOM-SM Darstellung gewonnen, bei der die n Referenzmesswerte 21 auf n Referenzvektoren rtj projiziert werden. Auch mit den wiederkehrend erfassten Messwerten 2 wird ein neuronales Netz erstellt, welches zu jedem Messzeitpunkt ti des Analysezeitraumes neu gebildet wird. Die Messwerte zu den Messzeitpunkten ti des Analysezeitraums werden dabei jeweils auf einen Messvektor xti projiziert. Dabei handelt es sich bei den projizierten Referenzvektoren rtj und den Messvektoren xti zu jedem Zeitpunkt tj und ti um Vektoren in einem zweidimensionalen Vektorraum VR.

Die Referenzvektoren rtj und die Messvektoren xti werden dabei unmittelbar anschließend zur in-line Erfassung der Messvariablen zum Zeitpunkt ti verglichen. Zu jedem Zeitpunkt ti wird anhand der Referenzvektoren rtj und der Messvektoren xti der Wert des Kerndichteschätzers bestimmt, wobei hier als Wahrscheinlichkeitsdichtefunktion PDF diejenige einer Normalverteilung verwendet wird:

$$p_h(xti) = \frac{1}{nh^d} \sum_{j=1}^{n} 2\pi^{-d/2} \cdot exp^{\frac{-(xti-rtj)^2}{2h^2}}$$

Die vorgebbare Fensterbreite h ist dabei jeweils abgeschätzt zu:

$$h = 1.06 \cdot in \qquad , \frac{IQ4R}{1.34} \Big\} \cdot n^{\frac{-1}{5}}$$

Gleichzeitig wird zu jedem Messzeitpunkt ti der euklidische Abstand zu dem Vektor des vorherigen Messzeitpunktes tk berechnet: |xti-xtkl. Bei den Messzeitpunkten tk und ti handelt es sich bei dieser Ausführung um direkt aufeinanderfolgende Messzeitpunkte (k=i-1). Andere Ausführungen, wobei k=i-2, i-3,..., etc. sind möglich.

Basierend auf einer Kombination des Kerndich-

teschätzers $p_h(xti)$ mit dem euklidischen Abstand |xti-xtk| wird dann eine Warnstufe ermittelt. In diesem Ausführungsbeispiel gibt es vier Warnstufen WO (keine Warnung), sowie W1,W2,W3 (erhöhte Warnstufen). Zu den Zeitpunkten ti, an denen eine Warnstufe oberhalb WO vorliegt, wird dann zusätzlich noch der Beitrag der Messvariablen MV1,MV2, MV3, MV4 zu der Warnstufe angezeigt, sowie der Beitrag des Kerndichteschätzers $p_h(xti)$, hier auch bezeichnet als MV_PZ, sowie des euklidischen Abstands Ixti-xtkl, hier auch bezeichnet als MV_ED.

## Bezugszeichenliste

| | |
|---|---|
| 1 | Fluid |
| 2 | Messwerte |
| 21 | Referenzmesswerte |
| 3 | Messgröße |
| 4 | Messstelle |
| 5 | Referenzdatensatz |
| 6 | Warnsystem |
| 7 | Messanordnung |
| 70 | übergeordnete Einheit |
| 71,72,73,... | in-line Messgeräte |
| 8 | Computerprogrammprodukt |
| 91 | Server |
| 92 | Cloud |
| 93 | Datenverbindung |
| 94 | Datenbank |

| | |
|---|---|
| RZ,RZ' | Referenzzustände |
| MV1,MV2,MV3,... | Messvariablen |
| tj, j=1,...,n | Zeitpunkte der Erfassung von 21 |
| ti | Zeitpunkt der Erfassung von 2 |
| tk | ein ti vorangehender Zeitpunkt der Erfassung von 2 |
| PDF | Wahrscheinlichkeitsdichtefunktion |
| d | reduzierte Dimension |
| xti | Messvektor |
| rtj j=1,...,n | Referenzvektoren |
| VR | Vektorraum |
| h | vorgebbare Fensterbreite |
| $p_h(xti)$ | Kerndichteschätzer für xti der vorgebbaren Fensterbreite h |
| $\sigma$ | Standardabweichung |
| IQR | Abstand von Quantilen |
| IQ4R | Interquartilabstand |
| Ka1,Ka2,... | Kategorien erster Art |
| Kb1,Kb2,... | Kategorien zweiter Art |
| G1,... | erster Grenzwert |
| G2,... | zweiter Grenzwert |
| WO,W1,... | Warnstufen |

## Patentansprüche

**1.** Verfahren zur automatisierten in-line Erkennung von Abweichungen eines Ist-Zustands eines in einem Prozess befindlichen Fluids (1) von einem Referenzzustand (RZ) des Fluids (1), wobei eine kombinierte Auswertung von im Wesentlichen gleichzeitig erfassten Messwerten (2) bezüglich zumindest dreier Messvariablen (MV1,MV2,MV3;...) erfolgt,

wobei es sich bei den Messvariablen (MV1,MV2,MV3;...) um unterschiedliche Messgrößen (3) des Fluides (1) und/oder um eine an unterschiedlichen Messstellen (4) gemessene Messgröße (3) des Fluids (1) handelt, um Veränderungen des Fluids unmittelbar zu erkennen, zu bewerten und ggf. anzuzeigen, wobei das Verfahren zumindest die folgenden Verfahrensschritte umfasst:

a) Erstellung eines Referenzdatensatzes (5) mittels folgender Schritte:

- Wiederkehrende in-line Erfassung zu n verschiedenen Zeitpunkten (tj, j=1,...,n) von jeweils im Wesentlichen gleichzeitig erfassten Referenzmesswerten (21) bezüglich der zumindest drei Messvariablen (MV1,MV2,MV3;...), wobei sich das Fluid (1) bei der Erfassung der Referenzmesswerte (21) in dem Referenzzustand (RZ) befindet; wobei vorzugsweise für den Fall von Trink- und/oder Rohwasser als das Fluid an ca. n=1000 Messzeitpunkten $t_j$ erfasste Referenzmesswerte erfasst werden;
- Speichern der Referenzmesswerte (21);
- Anordnung der Referenzmesswerte (21) mittels eines neuronalen Netzes und Projektion in einen Vektorraum (VR) mit einer reduzierten Dimension (d), wobei die reduzierte Dimension (d) kleiner als die Anzahl der Messvariablen (MV1,MV2,MV3;...) ist, so dass die zu dem Zeitpunkt tj erfassten Referenzmesswerte (21) jeweils auf einen Referenzvektor (rtj) des Vektorraumes (VR) abgebildet werden:
- Speichern der n Referenzvektoren rtj

b) In-line Messung, umfassend:

- In-line Erfassung von jeweils im Wesentlichen gleichzeitig erfassten Messwerte (2) bezüglich der zumindest drei Messvariablen (MV1,MV2,MV3;...) des Fluids (1) zu einem Zeitpunkt ti;
- Speichern der Messwerte (2);

c) Wiederkehrende Durchführung des Verfahrensschrittes b), wobei die Messwerte zu dem Zeitpunkt ti und die Messwerte zu allen dem Zeitpunkt ti vorangehenden Zeitpunkten (t1, ...,ti-1) mittels eines neuronalen Netzes wiederkehrend angeordnet und in den Vektorraum der reduzierten Dimension d projiziert werden, so dass die zu dem Zeitpunkt ti erfassten Messwerte auf einen Messvektor xti den Vektorraumes abgebildet werden;
d) Speichern des Messvektors xti;
e) Vergleich des Messvektors xti mit den n Referenzvektoren rtj mittels eines Kerndichteschätzers $p_h(xti)$ einer vorgebbaren Fensterbreite (h)

$$p_h(xti) = \frac{1}{nh^d} \sum_{j=1}^{n} f\left(\frac{|xti - rtj|}{h}\right)$$

wobei $f\left(\frac{|xti-rtj|}{h}\right)$ eine Wahrscheinlichkeitsdichtefunktion (PDF) ist;
f) Erstellung einer Bewertung für den Zeitpunkt ti zu hinsichtlich einer Abweichung des Ist-Zustands von dem Referenzzustand (RZ), basierend auf dem Wert des Kerndichteschätzers $p_h (xti)$,

wobei die wiederkehrende Durchführung des Verfahrensschrittes b) derart wiederkehrend durchgeführt wird, dass eine im Wesentlichen permanente und adaptive in-line Überwachung des Ist-Zustands des Fluids (1) ermöglicht ist.

2. Verfahren nach Anspruch 1,

wobei die Bildung des neuronalen Netzes und die Projektion auf Basis einer selbstorganisierenden Karte bzw. Kohonenkarte erfolgt, und wobei bei der Projektion die Sammon'sche Fehlerfunktion verwendet wird und/oder wobei die reduzierte Dimension (d) größer als eins ist, vorzugsweise zwei.

3. Verfahren nach zumindest einem der Ansprüche 1 oder 2, wobei die Wahrscheinlichkeitsdichtefunktion (PDF) die Wahrscheinlichkeitsdichtefunktion (PDF) einer Normalverteilung ist:

$$f\left(\frac{|xti - rtj|}{h}\right) = 2\pi^{-d/2} \cdot exp^{\frac{-(xti-rtj)^2}{2h^2}}$$

**4.** Verfahren nach zumindest einem der Ansprüche 1 bis 3
wobei die vorgebbare Fensterbreite (h) mittels der Standardabweichung ($\sigma$) der Verteilung der Referenzmesswerte (21) und/oder einem Abstand von Quantilen (IQR) der Referenzmesswerte (21) abgeschätzt wird.

**5.** Verfahren nach zumindest einem der Ansprüche 1 bis 4,

wobei die Bewertung des Fluides (1) zu dem Zeitpunkt ti hinsichtlich der Abweichung des Ist-Zustands vom Referenzzustand (RZ) mittels einer Einteilung in zumindest zwei verschiedene Kategorien erster Art (Ka1,Ka2,...) erfolgt,
und wobei die Einteilung davon abhängt, ob der Kerndichteschätzer $p_h(xti)$ einen ersten oberen und/oder unteren vorgebbaren Grenzwert (G1,...) über-/unterschreitet.

**6.** Verfahren nach zumindest einem der Ansprüche 1 bis 5,

wobei bei der Bewertung des Ist-Zustands des Fluides (1) für den Zeitpunkt ti der Unterschied zwischen dem Messvektor xti mit einem zweiten Messvektor xtk berücksichtigt wird,
wobei der zweite Messvektor xtk zu einem dem Zeitpunkt ti vorangehenden Zeitpunkt tk gehört.

**7.** Verfahren nach Anspruch 6,
wobei bei der Bewertung des Ist-Zustands des Fluides (1) zu dem Zeitpunkt ti eine Einteilung in zumindest zwei verschiedene Kategorien zweiter Art (Kb1,Kb2, ...) erfolgt, und wobei die Einteilung davon abhängt, ob der Unterschied zwischen dem Messvektor xti und dem zweiten Messvektor xtk einen zweiten oberen und/oder unteren vorgebbaren Grenzwert (G2,...) über-/unterschreitet.

**8.** Verfahren nach Anspruch 5 und 7,

wobei ein Warnsystem (6) mit zumindest zwei Warnstufen (W0,W1,...) zur Anzeige der Abweichung des Ist-Zustands vom Referenzzustand (RZ) und des Unterschiedes zwischen dem Messvektor xti und dem Vektor xtk verwendet wird,
und wobei das Warnsystem (6) auf einer Kombination der Kategorien erster Art (Ka1,Ka2,...) und der Kategorien zweiter Art (Kb1,Kb2,...) basiert.

**9.** Verfahren nach zumindest einem der Ansprüche 1 bis 8,

wobei das Warnsystem (6) zum Zeitpunkt ti den Beitrag jeder Messvariable (MV1,MV2,MV3;...) zu der Warnstufe (W0,W1,...) anzeigt,
und/oder
wobei das Warnsystem (6) zum Zeitpunkt ti den Beitrag Kategorien erster Art (Ka1,Ka2,...) und der Kategorien zweiter Art (Kb1,Kb2, ...) zu der Warnstufe (W0,W1,...) anzeigt.

**10.** Verfahren nach zumindest einem der Ansprüche 1 bis 9,

wobei der Verfahrensschritt a) für zumindest zwei unterschiedliche Referenzzustände (RZ,RZ') des Fluides (1) durchgeführt wird,
und wobei vor der Durchführung der Verfahrensschritte c)-f) der folgende Verfahrensschritt a2) durchgeführt wird,
a2) es wird einer der zumindest zwei unterschiedlichen Referenzzustände (RZ;RZ') ausgewählt;
wobei die Verfahrensschritte c)-f) anschließend zu dem Verfahrensschritt a2) jeweils in Bezug zu den jeweils ausgewählten Referenzzustand (RZ;RZ') durchgeführt werden.

**11.** Verfahren nach zumindest einem der Ansprüche 1 bis 10,
wobei es sich bei dem Fluid (1) um Roh- und/oder Trinkwasser handelt.

**12.** Verfahren nach zumindest einem der Ansprüche 1 bis 11,

wobei die zumindest zwei unterschiedlichen Referenzzustände (RZ,RZ',...) dadurch definiert sind,
ob das Roh- und/oder Trinkwasser einem Pumpbetrieb unterliegt oder nicht,
und/oder
wobei die zumindest zwei unterschiedlichen Referenzzustände (RZ,RZ',...) dadurch definiert sind,
ob das Roh- und/oder Trinkwasser aus unterschiedlichen Quellen, Seewasseranlagen und/oder Grundwasserleitern stammt.

**13.** Verfahren nach zumindest einem der Ansprüche 1 bis 12,
wobei die Messgrößen (3) ausgewählt sind aus der Gruppe der folgenden Messgrößen (3):

- Füllstand, Temperatur, Trübung, Sauerstoffgehalt, elektrische Leitfähigkeit, Druck, Redoxpotential, Durchfluss, pH-Wert, spektraler Absorptionskoeffizient.

**14.** Warnsystem zur automatisierten in-line Erkennung von Abweichungen eines Ist-Zustands eines Fluides (1) von einem Referenzzustand (RZ) des Fluides (1),

mit einer Messanordnung (7), aufweisend zumindest drei in-line Messgeräte (71,72,73,...) und
mit einem Computerprogrammprodukt (8), wobei die in-line Messgeräte (71,72,73,...) und das Computerprogrammprodukt (8) dazu ausgestaltet sind, das Verfahren nach zumindest einem der Ansprüche 1 bis 13 auszuführen.

**15.** Warnsystem nach Anspruch 14,

wobei das Computerprogrammprodukt (8) dazu ausgestaltet ist,
das Speichern und/oder die Verarbeitung der Referenzmesswerte (21) und/oder der Messwerte (2) zumindest teilweise auf zumindest einem Server (91) und/oder in einer Cloud (92) auszuführen.

## Claims

1. A method for automated inline detection of deviations of an actual state of a fluid (1) currently in a process from a reference state (RZ) of the fluid (1), wherein a combined evaluation of measured values essentially recorded at the same time (2) is performed with regard to at least three measurement variables (MV1, MV2, MV3;...), wherein the measurement variables (MV1, MV2, MV3; ...) are different measured values (3) of the fluid (1) and/or one measured variable (3) of the fluid (1) taken at various measuring points (4) in order to directly identify, evaluate and, where applicable, also display changes in the fluid, wherein the method comprises at least the following process steps:

   a) Definition of a reference data set (5) using the following steps:

   - Recurring inline recording at n different points in time (tj, j=1,...,n) of reference measured values (21), each taken essentially at the same time, with regard to the at least three measurement variables (MV1, MV2, MV3;...), wherein the fluid (1) is in the reference state (RZ) when recording the reference measured values (21); wherein preferentially for the case of drinking water and/or raw water the fluid is measured at approximately n=1000 measurement times $t_j$ to determine the reference measured values;
   - Saving of the reference measured values (21);
   - Arrangement of the reference measured values (21) using a neural network and projection into a vector space (VR) with one reduced dimension (d), wherein the reduced dimension (d) is smaller than the number of measurement variables (MV1, MV2, MV3; ...), so that the reference measured values (21) recorded at the time $t_j$ are each shown on a reference vector (rtj) in the vector space (VR):
   - Saving of the n reference vectors (rtj)

   b) Inline measurement, comprising:

   - Inline recording of measured values that are each essentially recorded at the same time (2) with regard to the at least three measurement variables (MV1, MV2, MV3; ...) of the fluid at a time ti;
   - Saving of the measured values (2);

   c) Recurring execution of process step b), wherein the measured values at the time ti and the measured values for all points in time preceding the time ti (t1, ... ,ti-1) are arranged on a repeated basis using a neural network and projected into the vector space of the reduced dimension d, so that the measured values recorded at the time ti are shown on a measurement vector xti in the vector space;
   d) Saving of the measurement vector xti;
   e) Comparison of the measurement vector xti with the n reference vectors rtj using a kernel density estimator $P_h$(xti) with a definable window width (h)

$$p_h(xti) = \frac{1}{nh^d} \sum_{j=1}^{n} f\left(\frac{|xti - rtj|}{h}\right)$$

   wherein f (|xti rtj|) is a probability density function (PDF);
   f) Production of an assessment for the time ti with regard to a variance of the actual state from the reference state (RZ), based on the value of the kernel density estimator $P_h$(xti),

   wherein the recurring execution of process step b) is performed on a recurring basis in such a way that an essentially permanent and adaptive inline monitoring of the actual state of the fluid (1) is facilitated.

2. A method according to claim 1,

   wherein the neural network is formed and the projection performed on the basis of a self-organizing map,
   and wherein Sammon's error function is used for the projection
and/or
wherein the reduced dimension (d) is greater than one, preferably two.

3. A method according to at least one of the claims 1 or 2,
wherein the probability density function (PDF) is the probability density function (PDF) of a normal distribution:

$$f\left(\frac{|xti - rtj|}{h}\right) = 2\pi^{-d/2} \cdot exp^{\frac{-(xti-rtj)^2}{2h^2}}$$

4. A method according to at least one of the claims 1 to 3,
wherein the definable window width (h) is estimated using standard deviation ($\sigma$) of the distribution of the reference measured values (21) and/or a spacing of quantiles (IQR) of the reference measured values (21).

5. A method according to at least one of the claims 1 to 4,

wherein the assessment of the fluid (1) at the time ti is performed with regard to the deviation of the actual state from the reference state (RZ) with classification into at least two different categories of the first type (Ka1, Ka2,...),
and wherein the classification is based on whether the kernel density estimator $P_h$(xti) exceeds or falls below a first upper and/or lower definable limit value (G1,...).

6. A method according to at least one of the claims 1 to 5,
wherein the difference between the measurement vector xti and a second measurement vector xtk is taken into account when assessing the actual state of the fluid (1) for the time ti, wherein the second measurement vector xtk belongs to a point in time preceding the time ti.

7. A method according to claim 6,
wherein a classification into at least two different categories of the second type (Kb1, Kb2, ...) is performed when assessing the actual state of the fluid (1) at the time ti, and wherein the classification is based on whether the difference between the measurement vector xti and the second measurement vector xtk exceeds or falls below a second upper and/or lower definable limit value (G2,...).

8. A method according to claims 5 and 7,

wherein a warning system (6) with at least two warning levels (W0, W1,...) is used for displaying the deviation of the actual state from the reference state (RZ), as well as the difference between the measurement vector xti and the vector xtk,
and wherein the warning system (6) is based on a combination of the categories of the first type (Ka1, Ka2,...) and the categories of the second type (Kb1, Kb2,...).

9. A method according to at least one of the claims 1 to 8,

wherein the warning system (6) at the time ti displays the contribution of each measurement variable (MV1, MV2, MV3; ...) to the warning level (W0, W1,...),
and/or
wherein the warning system (6) at the time ti displays the contribution of categories of the first type (Ka1, Ka2,...) and categories of the second type (Kb1, Kb2, ...) to the warning level (W0, W1,...).

10. A method according to at least one of the claims 1 to 9,

wherein the process step a) is performed for at least two different reference states (RZ, RZ') of the fluid (1),
and wherein the following process step a2) is performed prior to carrying out process steps c) to f),
wherein one of the at least two different reference states (RZ; RZ') is selected;
wherein the process steps c) to f) are then each performed for the process step a2) in reference to the reference state selected in each case (RZ; RZ').

11. A method according to at least one of the claims 1 to 10,
wherein the fluid (1) is raw water and/or drinking water.

12. A method according to at least one of the claims 1 to 11,

wherein the at least two different reference states (RZ, RZ',...) are defined by whether the raw water and/or drinking water is pumped or not, and/or
wherein the at least two different reference states (RZ, RZ',...) are defined by whether the raw water and/or drinking water comes from different sources, seawater plants, and/or aquifers.

**13.** A method according to at least one of the claims 1 to 12,

wherein the measured values (3) are selected from the group of the following measured values (3):

- Fill level, temperature, turbidity, oxygen content, electrical conductivity, pressure, redox potential, flow, pH value, spectral absorption coefficient.

**14.** A warning system for automated inline detection of deviations of an actual state of a fluid (1) from a reference state (RZ) of the fluid (1), with a measurement arrangement (7) that exhibits at least three inline measurement devices (71,72,73, ...) and a computer program product (8), wherein the inline measurement devices (71, 72, 73, ...) and the computer program product (8) are configured to execute the method according to at least one of the claims 1 to 13.

**15.** A warning system according to claim 14, wherein the computer program product (8) is configured to save and/or process the reference measured values (21) and/or the measured values (2) at least in part on at least one server (91) and/or in a cloud (92).

## Revendications

**1.** Procédé destiné à la détection automatisée en ligne d'écarts d'un état réel d'un fluide (1) se trouvant dans un process par rapport à un état de référence (RZ) du fluide (1), une évaluation combinée de valeurs mesurées (2) saisies pour l'essentiel simultanément concernant au moins trois variables de mesure (MV1, MV2, MV3, ...) étant effectuée,

les variables de mesure (MV1, MV2, MV3, ...) étant des grandeurs de mesure (3) différentes du fluide (1) et/ou une grandeur de mesure (3) du fluide (1) mesurée en différents points de mesure (4), afin de détecter, d'évaluer et, le cas échéant, d'afficher directement des changements du fluide,
lequel procédé comprend au moins les étapes suivantes :

a) Création d'un bloc de données de référence (5) au moyen des étapes suivantes :

- Saisie en ligne répétée à n moments différents (tj, j=1, ...,n) de valeurs mesurées de référence (21) saisies à chaque fois pour l'essentiel simultanément concernant les au moins trois variables de mesure (MV1, MV2, MV3, ...), le fluide (1) se trouvant dans l'état de référence (RZ) lors de la saisie des valeurs mesurées de référence (21) ;
procédé pour lequel on saisit de préférence, dans le cas de l'eau potable et/ou de l'eau brute en tant que fluide, des valeurs mesurées de référence à env. n=1000 instants de mesure $t_j$;
- Mémorisation des valeurs mesurées de référence (21) ;
- Disposition des valeurs mesurées de référence (21) au moyen d'un réseau neuronal et projection dans un espace vectoriel (VR) avec une dimension réduite (d), la dimension réduite (d) étant inférieure au nombre des variables mesurées (MV1, MV2, MV3, ...), de sorte que les valeurs mesurées de référence (21) saisies à l'instant tj sont reproduites respectivement sur un vecteur de référence (rtj) de l'espace vectoriel (VR) :
- Mémorisation des n vecteurs de référence rtj

b) Mesure en ligne, comprenant :

- Saisie en ligne de valeurs mesurées (2) respectivement saisies de manière pour l'essentiel simultanée concernant les au moins trois variables de mesure (MV1, MV2, MV3, ...) du fluide (1) en un instant ti ;
- Mémorisation des valeurs mesurées (2) ;

c) Exécution répétitive de l'étape de procédé b), les valeurs mesurées à l'instant ti et les valeurs mesurées à tous les instants (t1, ..., ti-1) précédant l'instant ti étant disposées de manière répétitive au moyen d'un réseau neuronal et projetées dans l'espace vectoriel de dimension d réduite, de sorte que les valeurs mesurées saisies à l'instant ti sont reproduites sur un vecteur de mesure xti de l'espace vectoriel ;
d) Mémorisation du vecteur de mesure xti ;
e) Comparaison du vecteur de mesure xti avec les n vecteurs de référence rtj au moyen d'un estimateur de densité par noyau $p_h(xti)$ d'une largeur de fenêtre (h) pouvant être prédéfinie

$$p_h(xti) = \frac{1}{nh^d} \sum_{i=1}^{n} f\left(\frac{|xti - rtj|}{h}\right)$$

où $f\left(\dfrac{|xti-rtj|}{h}\right)$ est une fonction de densité de probabilité (PDF) ;

f) Établissement d'une évaluation pour l'instant ti concernant un écart de l'état réel par rapport à l'état de référence (RZ), sur la base de la valeur de l'estimateur de densité par noyau $p_h$ (xti),

l'exécution de l'étape de procédé b) étant effectuée de manière répétitive de telle sorte qu'une surveillance en ligne pour l'essentiel permanente et adaptative de l'état réel du fluide (1) est rendue possible.

2. Procédé selon la revendication 1,

pour lequel la formation du réseau neuronal et la projection s'effectuent sur la base d'une carte auto-organisatrice ou d'une carte de Kohonen, et pour lequel la fonction d'erreur de Sammon est utilisée lors de la projection et/ou la dimension réduite (d) étant supérieure à un, de préférence à deux.

3. Procédé selon au moins l'une des revendications 1 ou 2,
pour lequel la fonction de densité de probabilité (PDF) est la fonction de densité de probabilité (PDF) d'une distribution normale :

$$f\left(\frac{|xti-rtj|}{h}\right) = 2\pi^{-d/2} \cdot exp^{\frac{-(xti-rtj)^2}{2h^2}}$$

4. Procédé selon au moins l'une des revendications 1 à 3,
pour lequel la largeur de fenêtre (h) pouvant être prédéfinie est estimée au moyen de l'écart-type ($\sigma$) de la distribution des valeurs mesurées de référence (21) et/ou d'une distance de quantiles (IQR) des valeurs mesurées de référence (21).

5. Procédé selon au moins l'une des revendications 1 à 4,

pour lequel l'évaluation du fluide (1) à l'instant ti en ce qui concerne l'écart de l'état réel par rapport à l'état de référence (RZ) s'effectue au moyen d'une classification en au moins deux catégories différentes de premier type (Ka1, Ka2, ...),
et pour lequel la classification dépend du fait que l'estimateur de densité par noyau $p_h$ (xti) est supérieur/inférieur à une première valeur limite supérieure et/ou inférieure (G1, ...) pouvant être prédéfinie.

6. Procédé selon au moins l'une des revendications 1 à 5,

pour lequel l'évaluation de l'état réel du fluide (1) pour l'instant ti tient compte de la différence entre le vecteur de mesure xti et un deuxième vecteur de mesure xtk,
le deuxième vecteur de mesure xtk appartenant à un instant tk précédant l'instant ti.

7. Procédé selon la revendication 6,

pour lequel, lors de l'évaluation de l'état réel du fluide (1) à l'instant ti, on effectue une classification en au moins deux catégories différentes de deuxième type (Kb1, Kb2, ...),
et pour lequel la classification dépend du fait que la différence entre le vecteur de mesure xti et le deuxième vecteur de mesure xtk est supérieure/inférieure à une deuxième valeur limite supérieure et/ou inférieure (G2, ...) pouvant être prédéfinie.

8. Procédé selon les revendications 5 et 7,

pour lequel un système d'alerte (6) avec au moins deux niveaux d'alerte (W0, W1, ...) est utilisé pour indiquer l'écart de l'état réel par rapport à l'état de référence (RZ) et la différence entre le vecteur de mesure xti et le vecteur xtk, et pour lequel le système d'alerte (6) est basé sur une combinaison des catégories de premier type (Ka1, Ka2, ...) et des catégories de deuxième type (Kb1, Kb2, ...).

9. Procédé selon au moins l'une des revendications 1 à 8,

pour lequel le système d'alerte (6) indique à l'instant ti la contribution de chaque variable de mesure (MV1, MV2, MV3, ...) au niveau d'alerte (W0, W1, ...),
et/ou
pour lequel le système d'alerte (6) indique à l'instant ti la contribution des catégories de premier type (Ka1, Ka2, ...) et des catégories de deuxième type (Kb1, Kb2, ...) au niveau d'alerte (W0, W1, ...).

10. Procédé selon au moins l'une des revendications 1 à 9,

pour lequel l'étape de procédé a) est mise en oeuvre pour au moins deux états de référence différents (RZ, RZ') du fluide (1), et
pour lequel, avant la mise en oeuvre des étapes de procédé c) à f), l'étape de procédé a2) suivante est mise en oeuvre,

a2) l'un des au moins deux états de référence différents (RZ, RZ') est sélectionné ;

les étapes de procédé c) à f) étant exécutées à la suite de l'étape de procédé a2) respectivement par rapport à l'état de référence sélectionné (RZ, RZ').

11. Procédé selon au moins l'une des revendications 1 à 10,

pour lequel le fluide (1) est de l'eau brute et/ou de l'eau potable.

12. Procédé selon au moins l'une des revendications 1 à 11,

pour lequel les au moins deux états de référence différents (RZ, RZ', ...) sont définis par le fait que l'eau brute et/ou l'eau potable est soumise ou non à un pompage,
et/ou
pour lequel les au moins deux états de référence différents (RZ, RZ', ...) sont définis par le fait, que l'eau brute et/ou l'eau potable provient de sources, d'installations d'eau de mer et/ou d'aquifères différents.

13. Procédé selon au moins l'une des revendications 1 à 12,
pour lequel les grandeurs de mesure (3) sont choisies dans le groupe des grandeurs de mesure (3) suivant :

- Niveau, température, turbidité, teneur en oxygène, conductivité électrique, pression, potentiel Redox, débit, pH, coefficient d'absorption spectrale.

14. Système d'alerte destiné à la détection automatisée en ligne d'écarts d'un état réel d'un fluide (1) par rapport à un état de référence (RZ) du fluide (1),

avec un dispositif de mesure (7), lequel présente au moins trois appareils de mesure en ligne (71, 72, 73, ...),
et
avec un produit de programme informatique (8), les appareils de mesure en ligne (71, 72, 73, ...) et le produit de programme informatique (8) étant conçus pour exécuter le procédé selon au moins l'une des revendications 1 à 13.

15. Système d'alerte selon la revendication 14,
pour lequel le produit de programme informatique (8) est configuré pour effectuer le stockage et/ou le traitement des valeurs mesurées de référence (21) et/ou des valeurs mesurées (2) au moins en partie sur au moins un serveur (91) et/ou dans un cloud (92).

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **R. PAGE.** *Multivariate Analysis of Groundwater-Quality Time-Series Using Self-organizing Maps and Sammon's Mapping,* 2015
- Water Resources Management. 3957ff
- **R. PAGE ; P. HUGGENBERGER.** *Aqua & Gas,* 2015, (12), 28ff